# EUROPÄISCHE PATENTANMELDUNG

(11) **EP 0 722 942 A1**
(43) Veröffentlichungstag der Anmeldung: **24.07.1996**
(21) Anmeldenummer: 96100253.2
(22) Anmeldetag: 10.01.1996
(51) Int. Cl.: C07D 401/14, C07D 401/04, A61K 31/445, A61K 31/47

(54) **Indolpiperidin-Derivate**

(30) Priorität: 12.01.1995 DE 19500689
(71) Anmelder: MERCK PATENT GmbH, D-64293 Darmstadt (DE)
(72) Erfinder: März, Joachim, Dr., D-55128 Mainz. (DE); Greiner, Hartmut, Dr., D-64331 Weiterstadt (DE); Seyfried, Christoph, Dr., D-64342 Seeheim-Jugenheim (DE); Bartoszyk, Gerd, D-64331 Weiterstadt (DE)

(57) **Zusammenfassung**

Indolpiperidin-Derivate der Formel I 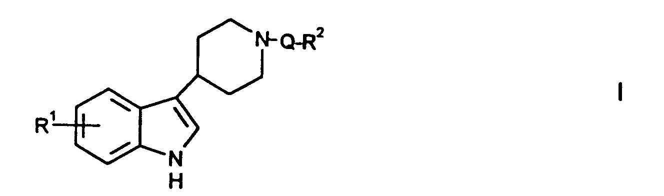
sowie deren physiologisch unbedenkliche Salze zeigen Wirkung auf das Zentralnervensystem, insbesondere Dopamin-agonistische oder Dopaminantagonistische Wirkung.

## Beschreibung

Die Erfindung betrifft Indolpiperidin-Derivate der Formel I 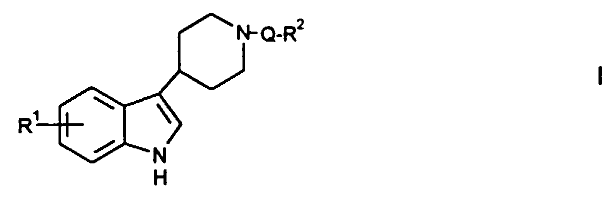 worin
- R¹: H, A, OH, OA, F, Cl, Br, I, CF₃, OCF₃, CN, COOH, CONH₂, CONHA, CONA₂ oder COOA,
- R²: -NH-CO-Ar, -NH-SO₂-Ar oder D,
- Q: CₘH₂ₘ oder -O-CₙH₂ₙ
- D: 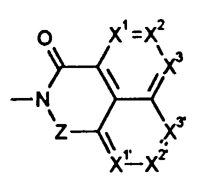
- X¹, X² und X³ sowie X^{1'}, X^{2'} und X^{3'}: jeweils unabhängig voneinander N oder CH, wobei die jeweiligen H-Atome auch durch einen Substituenten, ausgewählt aus einer Gruppe bestehend aus A, OA, F, Cl, Br, I, CF₃, OCF₃, CN, COOH oder COOA ersetzt sein können,
- Z: CO, SO₂ oder SO,
- A: Alkyl mit 1 bis 6 C-Atomen,
- Ar: unsubstituiertes oder ein- oder zweifach durch A, OA, F, Cl, Br, I, CF₃, OCF₃, CN, COOH oder COOA substituiertes 1-Naphthyl, wobei ebenfalls eine, zwei, drei oder vier CH-Gruppen durch N ersetzt sein können,
- m: 1, 2, 3 oder 4 und
- n: 1, 2 oder 3
bedeuten,
sowie deren physiologisch unbedenkliche Salze.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem serotonin-agonistische und -antagonistische Wirkungen. Sie hemmen die Serotonin-Wiederaufnahme sowie die Bindung von tritiierten Serotoninliganden an hippocampale Rezeptoren (Cossery et al., European J. Pharmacol. 140 (1987), 143-155). Insbesondere binden sie an 5HT₂- und D₂-Rezeptoren. Außerdem treten Veränderungen der DOPA-Akkumulation im Striatum und der 5-HTP-Akkumulation in N.raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt. Ebenso eignen sie sich als Prophylaxe und zur Bekämpfung der Folgen cerebraler Infarktgeschehen (Apoplexia cerebri), wie Schlaganfall und cerebraler Ischämien sowie zur Behandlung extrapyrimidal-motorischer Nebenwirkungen von Neuroleptika und des Morbus Parkinson. Ferner sind die Verbindungen zur Prophylaxe und zur Behandlung von Zwangsstörungen (obsessive compulsive disorders), Angstzuständen, Panikattacken, Depressionen, Psychosen, Schizophrenie, wahnhaften Zwangsvorstellungen, der Alzheimer Krankheit, Migräne, Anorexie, Bulimie und Drogenmißbrauch geeignet.

Verbindungen der Formel I und ihre physiologisch unbedenklichen Säureadditionssalze können daher z.B. als Arzneimittelwirkstoffe für Anxiolytika, Antidepressiva und/oder Antihypertonika und auch als Zwischenprodukte zur Herstellung anderer Arzneimittelwirkstoffe verwendet werden.

Gegenstand der Erfindung sind die Indolderivate der Formel I sowie ihre physiologisch unbedenklichen Säureadditionssalze.

Der Rest A bedeutet Alkyl mit 1, 2, 3, 4, 5 oder 6, insbesondere 1 oder 2 C-Atomen, vorzugsweise Methyl, ferner auch Ethyl, n-Propyl, Isopropyl, n-Butyl, Isobutyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy. NHA ist vorzugsweise Methylamino, ferner Ethylamino, n-Propylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Di-isopropylamino oder Di-n-butylamino.

Daraus resultierend bedeutet CO-NHA besonders bevorzugt N-Methylcarbamoyl oder N-Ethylcarbamoyl und CO-NA₂ vorzugsweise N,N-Dimethylcarbamoyl oder N,N-Diethylcarbamoyl.

Die Indolreste, die in 3-Position an den Piperidinrest gebunden sind, sind ansonsten unsubstituiert oder einfach substituiert. Die Substituenten befinden sich vorzugsweise in 5- oder 6-Stellung, ferner aber auch in der 4- und 7-Stellung.

Bevorzugte Substituenten R1 an dem Indolylrest sind beispielsweise CO₂H, CO₂CH₃, OCH₃, OH, F, CN oder CONH₂.

Q bedeutet vorzugsweise CₘH₂ₘ, wobei der Parameter m bevorzugt 2 oder 3, ist.

R² bedeutet bevorzugt 1- oder 2-Carboxamidonaphthyl, 2-, 3-, 4-, 5-, 6-, 7- oder 8-Carboxamidochinolinyl, 1-, 3-, 4-, 5-, 6-, 7- oder 8-Carboxamidoisochinolinyl, 1- oder 2-Sulfonamidonaphthyl, 2-, 3-, 4-, 5-, 6-, 7- oder 8-Sulfonamidochinolinyl, 1-, 3-, 4-, 5-, 6-, 7- oder 8-Sulfonamidoisochinolinyl, einen 2,3-Dihydro-1H-benz[de]isochinolin-1,3-dion-Rest oder einen 2,3-Dihydro-1H-benz[de]isochinolin-1-on-Rest. Die genannten Reste können auch substituiert sein. Bevorzugte Substituenten sind OA, CN, F, Cl oder Br.

Dementsprechend sind Gegenstand der Erfindung insbesondere diejenigen Verbindungen der Formel 1, in denen mindestens einer der genannten Reste eine der vorstehend angegebenen bevorzugten Bedeutungen hat. Einige bevorzugte Gruppen von Verbindungen können auch durch die nachstehenden Formeln Ia bis If ausgedrückt werden, die der Formel I entsprechen und worin die nicht näher bezeichneten Reste die bei der Formel I angegebene Bedeutung haben, worin jedoch
- in Ia: R¹, F, Cl, Br oder und R² Carboxamidonaphthyl oder Sulfonamidonaphthyl bedeuten;
- in Ib: R¹, F, Cl, Br oder I und R² Carboxamidochinolinyl oder Sulfonamidochinolinyl bedeuten;
- in Ic: R¹, F, Cl, Br I oder und R² einen 2,3-Dihydro-1H-benz[de]isochinolin-1,3-dion-Rest bedeuten;
- in Id: R¹ F und R² Carboxamidonaphthyl oder Sulfonamidonaphthyl bedeuten und R' sich in 4-Position des Indol-Restes befindet;
- in Ie: R¹ F und R² Carboxamidochinolinyl oder Sulfonamidochinolinyl bedeuten und R¹ sich in 5- oder 6-Position des Indol-Restes befindet;
- in If: R¹ F und R² einen 2,3-Dihydro-1H-benz[de]isochinolin-1,3-dion-Rest bedeuten und R¹ sich in 5- oder 6-Position des Indol-Restes befindet.

Insbesondere sind jedoch solche Verbindungen der Teilformeln lg sowie Iag bis Ifg bevorzugt, die den Teilformeln la bis lf und der Formel I entsprechen, worin jedoch zusätzlich Q -CH₂-CH₂-, -CH₂-CH₂-CH₂- oder O-CH₂-CH₂- bedeutet.

Gegenstand der Erfindung ist ferner ein Verfahren zur Herstellung von Indolpiperidin-Derivaten der Formel I gemäß Anspruch 1 sowie von deren Salzen, dadurch gekennzeichnet, daß man eine Verbindung der Formel II 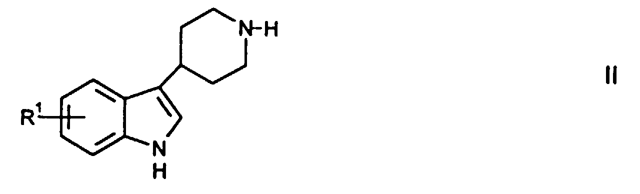 worin
R¹ die angegebene Bedeutung hat,
mit einer Verbindung der Formel III

R²-Q-L III

worin
- L, sofern Q für -CₘH₂ₘ- steht: Cl, Br, I, OH, O-CO-A', O-CO-Ph, O-SO₂-A', O-SO₂Ph oder eine andere reaktionsfähig veresterte OH-Gruppe oder leicht nucleophil substituierbare Abgangsgruppe bedeutet und
- L, sofern Q für -O-CₙH₂ₙ-: steht H, -CO-A', -CO-Ph, -SO2-A', -SO₂-Ph, wobei in beiden Fällen Ph für Phenyl oder Tolyl und A' für Alkyl oder Fluoralkyl stehen, oder eine andere reaktionsfähig veresterte OH-Gruppe oder leicht nucleophil substituierbare Abgangsgruppe bedeutet und
R² und Q die angegebenen Bedeutungen haben,
umsetzt,
oder daß man eine-Verbindung der Formel IV 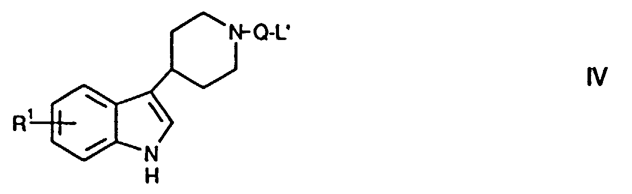 worin
- L': Cl, Br, I, OH, O-CO-A', O-COPh, O-SO₂-A', O-SO₂-Ph, wobei Ph für Phenyl oder Tolyl und A' für Alkyl oder Fluoralkyl stehen, oder eine andere reaktionsfähig veresterte OH-Gruppe oder leicht nucleophil substituierbare Abgangsgruppe bedeutet und
R¹ und Q die angegebenen Bedeutungen haben,
mit einer primären oder sekundären Aminoverbindung der Formel V

R²-H V

worin
R² die angegebene Bedeutung hat,
umsetzt,
oder daß man eine Verbindung der Formel VI 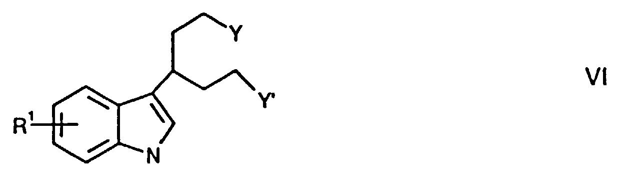 worin
- Y und Y': gleich oder verschieden sein können und Cl, Br, I, OH, O-CO-A', O-CO-Ph, O-SO₂-A', O-SO₂Ph, wobei Ph für Phenyl oder Tolyl und A' für Alkyl oder Fluoralkyl stehen, oder eine andere reaktionsfähig veresterte OH-Gruppe oder leicht nucleophil substituierbare Abgangsgruppe bedeutet und
R¹ die angegebene Bedeutung hat,
mit einem Amin der Formel VII

R²-Q-NH₂ VII

worin
R² und Q die angegebenen Bedeutungen haben,
umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C-und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls einen Rest R¹ in einen anderen Rest R¹ umwandelt, indem man z.B. eine OA-Gruppe unter Bildung einer OH-Gruppe spaltet und/oder eine CN-, COOH- oder COOA-Gruppe in eine andere funktionelle Gruppe umwandelt
und/oder daß man eine erhaltende Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg-Thieme-Verlag, Stuttgart; Organic Reactions, John Wiley & Sons, Inc. New York; J. March, Adv. Org. Chem., 3rd Ed. J. Wiley & Sons (1985) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel l umsetzt.

Eine bevorzugte Methode zur Herstellung von Verbindungen der Formel I besteht darin, daß man ein 3-(4-Piperidyl)-indol-Derivat der Formel II mit einer elektrophilen Verbindung der Formel III umsetzt.

Die Verbindungen der Formeln II und insbesondere III sind zum Teil bekannt; die nicht bekannten Verbindungen der Formeln II und III können leicht analog zu den bekannten Verbindungen hergestellt werden.

3-(4-Piperidyl)-indole der Formel II können z.B. durch Umsetzung von 4-Halogenpiperidinen, wobei Halogen vorzugsweise Chlor oder Brom bedeutet, mit Indol oder entsprechenden durch R1 substituierten Derivaten hergestellt werden. Diese Reaktionen sind an sich bekannt und werden unter üblichen Reaktionsbedingungen durchgeführt, wie sie aus der Literatur für die elektrophile Substitution an Indolen bekannt sind.

Verbindungen der Formel III können beispielsweise hergestellt werden, indem man eine Verbindung der Formel Ar-CONH₂, Ar-SO₂NH₂ oder D-H, wobei Ar und D die angegebenen Bedeutungen besitzen, mit einer Verbindung der Formel L-Q-L' unter den üblichen Bedingungen der N-Alkylierung umsetzt.

Primäre Alkohole der Formel III sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel III (Hal: Br, Cl). Die entsprechenden Sulfonytoxyverbindungen sind erhältlich aus den Alkoholen durch Umsetzung mit den entsprechenden Sulfonsäurechloriden.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander zur Reaktion bringen, gegebenenfalls im geschlossenen Rohr oder im Autoklaven. Es ist aber bevorzugt, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser.

Die Reaktionszeit liegt je nach den angewendeten Bedingungen zwischen einigen Minuten und 14 Tagen, die Reaktionstemperatur zwischen etwa 0 und 150 °C, normalerweise zwischen 20 und 130 °C.

In einigen Fällen kann der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin die Reaktion begünstigen. In anderen Fällen ist der Zusatz katalytischer Mengen einer Säure, vorzugsweise einer Mineralsäure wie z.B. HCI, günstig.

Ferner ist es möglich eine Verbindung der Formel I zu erhalten, indem man eine Verbindung der Formel V mit einem Indolderivat der Formel IV umsetzt.

Die Indole der Formel IV lassen sich durch die diversen, an sich bekannten Möglichkeiten der Indolsynthese, beispielsweise der Fischer-Indolsynthese, herstellen. Es ist außerdem besonders bevorzugt z.B. entsprechend substituierte 4-Halogenpiperidine mit durch R¹-substituierten Indolen zu Verbindungen der Formel IV umzusetzen.

Verbindungen der Formel V sind in der Regel bekannt und können z.B. durch Überführung der entsprechenden Carbonsäuren Ar-COOH oder Sulfonsäuren Ar-SO₂OH in die jeweiligen Amide hergestellt werden.

Die Umsetzung der Verbindungen IV und V verläuft nach Methoden, wie sie für die Reaktionen von Aminen oder Amiden mit elektrophilen Reaktionspartnern aus der Literatur bekannt sind. Die Komponenten können direkt ohne Gegenwart eines Lösungsmittels, miteinander umgesetzt werden, gegebenenfalls im geschlossenen Rohr oder im Autoklaven, unter Normaldruck oder unter erhöhtem Druck, wobei ein Inertgas wie z.B. N₂ zur Druckerhöhung zugeführt wird. Es ist aber auch möglich, die Verbindungen in Gegenwart eines inerten Lösungsmittels umzusetzen. Als Lösungsmittel eignen sich die zuvor bei der Umsetzung von II mit III genannten.

Die optimale Reaktionszeit liegt, je nach den gewählten Reaktionsbedingungen, zwischen einigen Minuten und 14 Tagen, die Reaktionstemperatur zwischen etwa 0° und 150°, üblicherweise zwischen 20° und 130°.

Weiterhin ist es möglich, daß man ein Indol-Derivat der Formel VI mit einem Amin der Formel VII umsetzt, um eine Verbindung der Formel I zu erhalten.

Indole der Formel VI können beispielsweise über eine elektrophile Substitution in 3-Position eines Indols durch Umsetzung mit einer Verbindung der Formel Y-(CH₂)₂-CH(Hal)-CH₂)₂-Y', worin Hal z.B. Cl oder Br bedeutet erhalten werden, während die Verbindungen der Formel VII durch Alkylierung der entsprechenden Amide, Sulfonamide oder Amine der Formel R²-H mit einer Verbindung der Formel L-Q-NH₂, wobei die NH₂-Gruppe zweckmäßigerweise durch an sich bekannte Aminoschutzgruppen blockiert wird, erhalten werden können.

Die Umsetzung der Verbindungen VI mit den Aminen VII verläuft nach Methoden, wie sie für die Alkylierung von Aminen aus der Literatur bekannt sind und zuvor, für die Umsetzung von Verbindung II mit Verbindung III, kurz beschrieben wurden.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Indolreste, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Weiterhin können Verbindungen, die sonst der Formel I entsprechen, aber anstelle eines oder mehrerer H-Atome eine oder mehrere solvolysierbare Gruppe(n) enthalten, zu den Verbindungen der Formel I solvolysiert, insbesondere hydrolysiert werden.

So können insbesondere 1-Acylindolderivate (entsprechend der Formel I, aber in 1-Stellung des Indol-Rests eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, beson-ders die Wasser enthaltenden Gemische. Eine Hydrolyse kann auch bereits beim Behandeln mit Wasser allein erfolgen, insbesondere in der Siedehitze.

Weiterhin kann man eine Verbindung der Formel I nach an sich bekannten Methoden in eine andere Verbindung der Formel I umwandeln.

Verbindungen der Formel I, worin das Indol-System z.B. durch COOA, CONH₂, CONHA, CONA₂ substituiert ist, können durch Derivatisierung entsprechender Carboxy-indol-3-yl-Verbindungen erhalten werden. Man kann z.B. die Säuren oder ihre reaktionsfähigen Derivate, wie z.B. ihre Säurehalogenide oder Anhydride mit entsprechenden Alkoholen oder Alkoholaten, unter Verwendung der an sich bekannten Methoden oder einer der zahlreichen Varianten, verestern. Femer ist es möglich, Säuren, Säurehalogenide oder Ester mit primären oder sekundären, aliphatischen oder cyclischen Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicydohexylcarbodiimid oder N-(3-Dimethylaminopropyl)-N-ethyl-carbodiimid, ferner Propanphosphonsäureanhydrid (vgl. Angew. Chemie 92, 129 (1980)), Diphenylphosphorylazid oder 2-Ethoxy-N-ethoxycarbonyt-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°. Anstelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind. Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

Weiterhin kann man cyan-substituierte Indol-3-yl-Reste zu Carboxy-indol-3-yl- oder Carbamido-indol-3-yl-Resten hydrolysieren.

Verbindungen der Formel I, in denen die Indol-Reste ein- oder zweifach durch O-Alkyl substituiert sind, können einer Etherspaltung unterzogen werden, wobei die entsprechenden Hydroxyderivate entstehen. ZB. kann man die Ethergruppen spalten durch Behandeln mit Dimethylsulfid-Bortribromid-Komplex, z.B. in Toluol, Ethem wie THF oder Dimethylsulfoxid, oder durch Verschmelzen mit Pyridin- oder Anilinhydrohalogeniden, vorzugsweise Pyridinhydrochlorid, bei etwa 150-250°.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure. Phosphorsäuren wie Orthophosphorsäure, Salpetersäure, Sulfaminsäure, ferner organische Säuren, im einzelnen aliphatische, alicydische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bemsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel 1 und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlenhydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung extrapyramidal-motorischer Nebenwirkungen von Neuroleptika, von Erkrankungen des Zentralnervensystems wie Spannungszuständen, Depressionen und/oder Psychosen und von Nebenwirkungen bei der Behandlung der Hypertonie (z.B. mit α-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide und zur Bekämpfung der Folgen cerebraler Infarktgeschehen (Apoplexia cerebri), wie Schlaganfall und cerebraler Ischämien.

Dabei werden die erfindungsgemäßen Substanzen in der Regel in Analogie zu bekannten, im Handel befindlichen Präparaten, z.B. Bromocriptin, Dihydroergocornin verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg je Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welche die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. In den Fällen, in denen die Substanzen zur Zersetzung neigen, werden ersatzweise die Rf-Werte angegeben.

### Beispiel 1

Man löst 1,2 g 2-(2-Chlorethyl)-2,3-dihydro-1H-benz[de]isochinolin-1,3-dion ("A") [erhältlich durch Umsetzung von 2,3-Dihydro-1H-benz[de]iso-chinolin-1,3-dion mit 1,2-Dichlorethan zu 2-(2-Chlor-ethyl)-2'3-dihydro-1H-benz[de]isochinolin-1,3-dion] sowie 1,0 g 4-(5-Fluor-indol-3-yl)-piperidin [erhältlich durch Umsetzung von N-BOC-4-Chlor-piperidin mit 5-Fluorindol und anschließende Abspaltung der Schutzgruppe] in 200 ml Acetonitril und rührt 8 Stunden bei Raumtemperatur. Nach üblicher Aufarbeitung erhält man 2-[2-(4-(5-Fluor-3-indolyl)-piperidino)-ethyl]-2,3-dihydro-1H-benz[*de*]isochinolin-1,3-dion, Tetrahydrat, F. 235°.

Analog erhält man durch Umsetzung von "A"
mit 4-(4-Fluor-indol-3-yl)-piperidin:
   2-[2-(4-(4-Fluor-3-indolyl)-piperidino)-ethyl]-2,3-dihydro-1H-benz[*de*]-isochinolin-1,3-dion, Rf = 0,82 (Ethylacetat/Methanol 3:1);
mit 4-(6-Fluor-indol-3-yl)-piperidin:
   2-[2-(4-(6-Fluor-3-indolyl)-piperidino)-ethyl]-2,3-dihydro-1H-benz[*de*]-isochinolin-1,3-dion, F. 264°;
mit 4-(5-Methoxy-indol-3-yl)-piperidin:
   2-[2-(4-(5-Methoxy-3-indolyl)-piperidino)-ethyl]-2,3-dihydro-1H-benz[*de*]isochinolin-1,3-dion;
mit 4-(6-Methoxy-indol-3-yl)-piperidin:
   2-[2-(4-(6-Methoxy-3-indolyl)-piperidino)-ethyl]-2,3-dihydro-1H-benz-[*de*]isochinolin-1,3-dion;
mit 4-(5-Trifluormethoxy-indol-3-yl)-piperidin:
   2-[2-(4-(5-Trifluormethoxy-3-indolyl)-piperidino)-ethyl]-2,3-dihydro-1H-benz[*de*]isochinolin-1,3-dion;
mit 4-(4-Cyan-indol-3-yl)-piperidin:
   2-[2-(4-(4-Cyan-3-indolyl)-piperidino)-ethyl]-2,3-dihydro-1H-benz[*de*]-isochinolin-1,3-dion;
mit 4-(6-Cyan-indol-3-yl)-piperidin:
   2-[2-(4-(6-Cyan-3-indolyl)-piperidino)-ethyl]-2,3-dihydro-1H-benz[*de*]-isochinolin-1,3-dion;
mit 4-(5-Cyan-indol-3-yl)-piperidin:
   2-[2-(4-(5-Cyan-3-indolyl)-piperidino)-ethyl]-2,3-dihydro-1 H-benz[*de*]-isochinolin-1,3-dion;
mit 4-(6-Trifluormethyl-indol-3-yl)-piperidin:
   2-[2-(4-(6-Trifluormethyl-3-indolyl)-piperidino)-ethyl]-2,3-dihydro-1H-benz[*de*]isochinolin-1,3-dion;
mit 4-(4-Trifluormethyl-indol-3-yt)-piperidin:
   2-[2-(4-(4-Trifluormethyl-3-indolyl)-piperidino)-ethyl]-2,3-dihydro-1H-benz[*de*]isochinolin-1,3-dion;
mit 4-(5-Methoxycarbonyl-indol-3-yl)-piperidin:
   2-[2-(4-(5-Methoxycarbonyl-3-indolyl)-piperidino)-ethyl]-2,3-dihydro-1H-benz[*de*]isochinolin-1,3-dion;
mit 4-(6-Methoxycarbonyl-indol-3-yl)-piperidin:
   2-[2-(4-(6-Methoxycarbonyl-3-indolyl)-piperidino)-ethyl]-2,3-dihydro-1H-benz[*de*]isochinolin-1,3-dion;
mit 4-(4-Methoxycarbonyl-indol-3-yl)-piperidin:
   2-[2-(4-(4-Methoxycarbonyl-3-indolyl)-piperidino)-ethyl]-2,3-dihydro-1H-benz[*de*]isochinolin-1,3-dion;
mit 4-(5-Chlor-indol-3-yl)-piperidin:
   2-[2-(4-(5-Chlor-3-indolyl)-piperidino)-ethyl]-2,3-dihydro-1H-benz[*de*]isochinolin-1,3-dion;
mit 4-(6-Chlor-indol-3-yl)-piperidin:
   2-[2-(4-(6-Chlor-3-indolyl)-piperidino)-ethyl]-2,3-dihydro-1H-benz[*de*]isochinolin-1,3-dion;
mit 4-(4-Chlor-indol-3-yl)-piperidin:
   2-[2-(4-(4-Chlor-3-indolyl)-piperidino)-ethyl]-2,3-dihydro-1H-benz[*de*]isochinolin-1,3-dion.

### Beispiel 2

Man kocht 0,8 g 2-[2-(4-(5-Methoxycarbonyl-3-indolyl)-piperidino)-ethyl]-2,3-dihydro-1H-benz[*de*]isochinolin-1,3-dion [erhältlich nach Beispiel 1] 0,5 Std. mit 100 ml 2n ethanolischer KOH, arbeitet wie üblich auf und erhält 2-[2-(4-(5-Carboxy-3-indolyl)-piperidino)-ethyl]-2,3-dihydro-1H-benz[*de*]isochinolin-1 ,3-dion.

Analog erhält man durch Verseifung der entsprechenden Ester ausgehend
von 2-[2-(4-(6-Methoxycarbonyl-3-indolyl)-piperidino)-ethyl]-2,3-dihydro-1H-benz[*de*]iso-chinolin-1,3-dion:
   2-[2-(4-(6-Carboxy-3-indolyl)-piperidino)-ethyl]-2,3-dihydro-1H-benz[*de*]iso-chinolin-1,3-dion;
von 2-[2-(4-(4-Methoxycarbonyl-3-indolyl)-piperidino)-ethyl]-2,3-dihydro-1H-benz[*de*]isochinolin-1,3-dion:
   2-[2-(4-(4-Carboxy-3-indolyl)-piperidino)-ethyl]-2,3-dihydro-1H-benz[*de*]isochinolin-1,3-dion.

### Beispiel 3

Analog Beispiel 1 erhält man ausgehend von 1-(2-Aminoethyl)-4-(5-fluorindol-3-yl)-piperidin [erhältlich durch Umsetzung von N-BOC-4-Chlorpiperidin mit 5-Fluor-indol, anschließende Abspaltung der Schutzgruppe, Umsetzung mit 1-Chlor-2-amino-ethan] und 8-Chinolinsulfonyl-chlorid ("B") nach üblicher Aufarbeitung das N-[2-(4-(5-Fluor-3-indolyl)-piperidino)-ethyl]-8-chinolinsulfonamid, Hydrochlorid-Hemihydrat F. 290°.

Analog erhält man durch Umsetzung von "B"
mit 1-(2-Aminoethyl)-4-(4-fluor-indol-3-yl)-piperidin:
   N-[2-(4-(4-Fluor-3-indolyl)-piperidino)-ethyl]-8-chinolinsulfonamid;
mit 1-(2-Aminoethyl)-4-(6-fluor-indol-3-yl)-piperidin:
   N-[2-(4-(6-Fluor-3-indolyl)-piperidino)-ethyl]-8-chinolinsulfonamid, Hydrochlorid, F. 276°;
mit 4-(5-Ethoxy-indol-3-yl)-piperidin:
   N-[2-(4-(5-Ethoxy-3-indolyl)-piperidino)-ethyl]-8-chinolinsulfonamid;
mit 4-(5-Methoxy-indol-3-yl)-piperidin:
   N-[2-(4-(5-Methoxy-3-indolyl)-piperidino)-ethyl]-8-chinolinsulfonamid;
mit 4-(6-Methoxy-indol-3-yl)-piperidin:
   N-[2-(4-(6-Methoxy-3-indolyl)-piperidino)-ethyl]- 8-chinolinsulfonamid;
mit 4-(5-Trifluormethoxy-indol-3-yl)-piperidin:
   N-[2-(4-(5-Trifluormethoxy-3-indolyl)-piperidino)-ethyl]-8-chinolinsulfonamid;
mit 4-(4-Cyan-indol-3-yl)-piperidin:
   N-[2-(4-(4-Cyan-3-indolyl)-piperidino)-ethyl]-8-chinolinsulfonamid;
mit 4-(6-Cyan-indol-3-yl)-piperidin:
   N-[2-(4-(6-Cyan-3-indolyl)-piperidino)-ethyl]-8-chinolinsulfonamid;
mit 4-(5-Cyan-indol-3-yl)-piperidin:
   N-[2-(4-(5-Cyan-3-indolyl)-piperidino)-ethyl]-8-chinolinsulfonamid;
mit 4-(6-Trifluormethyl-indol-3-yl)-piperidin:
   N-[2-(4-(6-Trifluormethyl-3-indolyl)-piperidino)-ethyl]-8-chinolinsulfonamid;
mit 4-(4-Trifluormethyl-indol-3-yl)-piperidin:
   N-[2-(4-(4-Trifluormethyl-3-indolyl)-piperidino)-ethyl]-8-chinolinsulfonamid;
mit 4-(5-Methoxycarbonyl-indol-3-yl)-piperidin:
   N-[2-(4-(5-Methoxycarbonyl-3-indolyl)-piperidino)-ethyl]-8-chinolinsulfonamid;
mit 4-(6-Methoxycarbonyl-indol-3-yl)-piperidin:
   N-[2-(4-(6-Methoxycarbonyl-3-indolyl)-piperidino)-ethyl]-8-chinolinsulfonamid;
mit 4-(4-Methoxycarbonyl-indol-3-yl)-piperidin:
   N-[2-(4-(4-Methoxycarbonyl-3-indolyl)-piperidino)-ethyl]-8-chinolinsulfonamid;
mit 4-(5-Chlor-indol-3-yl)-piperidin:
   N-[2-(4-(5-Chlor-3-indolyl)-piperidino)-ethyl]-8-chinolinsulfonamid;
mit 4-(6-Chlor-indol-3-yl)-piperidin:
   N-[2-(4-(6-Chlor-3-indolyl)-piperidino)-ethyl]-8-chinolinsulfonamid;
mit 4-(4-Chlor-indol-3-yl)-piperidin:
   N-[2-(4-(4-Chlor-3-indolyl)-piperidino)-ethyl]-8-chinolinsulfonamid.

### Beispiel 4

Analog Beispiel 2 erhält man durch Verseifung von N-[2-(4-(5-Methoxy-carbonyl-3-indolyl)-piperidino)-ethyl]-8-chinolinsulfonamid das N-[2-(4-(5-Carboxy-3-indolyl)-piperidino)-ethyl]-8-chinolinsulfonamid.

Analog erhält man durch Verseifung der entsprechenden Ester ausgehend
von N-[2-(4-(6-Methoxycarbonyl-3-indolyl)-piperidino)-ethyl]-8-chinolinsulfonamid:
   N-[2-(4-(6-Carboxy-3-indolyl)-piperidino)-ethyl]-8-chinolinsulfonamid;
von N-[2-(4-(4-Methoxycarbonyl-3-indolyl)-piperidino)-ethyl]-8-chinolinsulfonamid:
   N-[2-(4-(4-Carboxy-3-indolyl)-piperidino)-ethyl]-8-chinolinsulfonamid.

### Beispiel 5

2,1 g 2-[2-(4-(5-Carboxy-3-indolyl)-piperidino)-ethyl]-2,3dihydro-1H-benz[*de*]isochinolin-1,3-dion werden in 100 ml N-Methylpyrrolidin suspendiert. Anschließend fügt man 3,2 g 2-Chlor-1-methyl-pyridiniummethan-sulfonat hinzu und rührt bei Raumtemperatur 12 Stunden. In die entstandene Lösung leitet man bis zur Sättigung getrocknetes NH3-Gas ein und rührt erneut 10 Stunden. Nach üblicher Aufarbeitung erhält man 2-[2-(4-(5-Carbamoyl-3-indolyl)-piperidino)-ethyl]-2,3-dihydro-1H-benz[*de*]isochinolin-1,3-dion

Analog erhält man durch Amidierung der nachfolgenden Carbonsäuren:
aus 2-[2-(4-(6-Carboxy-3-indolyl)-piperidino)-ethyl]-2,3-dihydro-1H-benz[*de*]isochinolin-1,3-dion:
   2-[2-(4-(6-Carbamoyl-3-indolyl)-piperidino)-ethyl]-2,3dihydro-1H-benz[*de*]isochinolin-1,3-dion;
aus 2-[2-(4-(4-Carboxy-3-indolyl)-piperidino)-ethyl]-2,3-dihydro-1H-benz[*de*]isochinolin-1,3-dion:
   2-[2-(4-(4-Carbamoyl-3-indolyl)-piperidino)-ethyl]-2,3-dihydro-1H-benz[*de*]isochinolin-1,3-dion;
aus N-[2-(4-(5-Carboxy-3-indolyl)-piperidino)-ethyl]-8-chinolinsulfonamid:
   N-[2-(4-(5-Carbamoyl-3-indolyl)-piperidino)-ethyl]-8-chinolinsulfonamid;
aus N-[2-(4-(6-Carboxy-3-indolyl)-piperidino)-ethyl]-8-chinolinsulfonamid:
   N-[2-(4-(6-Carbamoyl-3-indolyl)-piperidino)-ethyl]-8-chinolinsulfonamid;
aus N-[2-(4-(4-Carboxy-3-indolyl)-piperidino)-ethyl]-8-chinolinsulfonamid:
   N-[2-(4-(4-Carbamoyl-3-indolyl)-piperidino)-ethyl]-8-chinolinsulfonamid.

### Beispiel 6

Eine Lösung von 3,9 g 2-[2-(4-(5-Carboxy-3-indolyl)-piperidino)-ethyl]-2,3-dihydro-1H-benz[*de*]isochinolin-1,3-dion in 250 ml DMF wird mit 1 g N-Methylmorpholin versetzt. Unter Rühren gibt man eine Lösung von zwei Äquivalenten tert.-Butylamin in 5 ml DMF, 1,3 g 1-Hydroxybenztriazol sowie eine Lösung von 1,9 g N-(3-Dimethylaminopropyl)-N'-ethyl-carbodiimid-hydrochlorid in 20 ml DMF hinzu. Man rührt 16 Stunden bei Raumtemperatur und dampft das Filtrat ein. Nach üblicher Aufarbeitung erhält man 2-[2-(4-(5-N-tert.-Butylcarbamoyl-3-indolyl)-piperidino)-ethyl]-2,3-dihydro-1H-benz[de]isochinolin-1,3-dion.

Analog erhält man durch Umsetzung mit tert.-Butylamin ausgehend
von 2-[2-(4-(6-Carboxy-3-indolyl)-piperidino)-ethyl]-2,3-dihydro-1H-benz-[*de*]isochinolin-1,3-dion:
   2-[2-(4-(6-N-tert.-Butyl-carbamoyl-3-indolyl)-piperidino)-ethyl]-2,3-dihydro-1H-benz[*de*]isochinolin-1,3-dion;
von 2-[2-(4-(4-Carboxy-3-indolyl)-piperidino)-ethyl]-2,3-dihydro-1H-benz-[*de*]isochinolin-1,3-dion:
   2-[2-(4-(4-N-tert.-Butyl-carbamoyl-3-indolyl)-piperidino)-ethyl]-2,3-dihydro-1H-benz[*de*]isochinolin-1,3-dion;
von N-[2-(4-(5-Carboxy-3-indolyl)-piperidino)-ethyl]-8-chinolinsulfonamid:
   N-[2-(4-(5-N-tert.-Butyl-carbamoyl-3-indolyl)-piperidino)-ethyl]-8-chinolinsulfonamid;
aus N-[2-(4-(6-Carboxy-3-indolyl)-piperidino)-ethyl]-8-chinolinsulfonamid:
   N-[2-(4-(6-N-tert.-Butyl-carbamoyl-3-indolyl)-piperidino)-ethyl]-8-chinolinsulfonamid;
aus N-[2-(4-(4-Carboxy-3-indolyl)-piperidino)-ethyl]-8-chinolinsulfonamid:
   N-[2-(4-(4-N-tert.-Butyl-carbamoyl-3-indolyl)-piperidino)-ethyl]-8-chinolinsulfonamid.

### Beispiel 7

Eine Gemisch von 1,6 g 2-[2-(4-(5-Methoxy-3-indolyl)-piperidino)-ethyl]-2,3-dihydro-1H-benz[*de*]isochinolin-1,3-dion [herstellbar nach Beispiel 1], 1,8 g Pyridinhydrochlorid sowie 50 ml Pyridin wird 3 Stunden gekocht. Man kühlt ab, dampft ein, arbeitet wie üblich auf und erhält 2-[2-(4-(5-Hydroxy-3-indolyl)-piperidino)-ethyl]-2,3-dihydro-1H-benz[*de*]isochinolin-1,3-dion.

Analog erhält man
aus 2-[2-(4-(6-Methoxy-3-indolyl)-piperidino)-ethyl]-2,3-dihydrn-1H-benz[*de*]isochinolin-1 ,3-dion:
   2-[2-(4-(5-Hydroxy-3-indolyl)-piperidino)-ethyl]-2,3-dihydro-1H-benz[*de*]isochinolin-1,3-dion ;
aus N-[2-(4-(5-Methoxy-3-indolyl)-piperidino)-ethyl]-8-chinolinsulfonamid:
   N-[2-(4-(5-Hydroxy-3-indolyl)-piperidino)-ethyl]-8-chinolinsulfonamid;
aus N-[2-(4-(6-Methoxy-3-indolyl)-piperidino)-ethyl]- 8-chinolinsulfonamid:
   N-[2-(4-(6-Hydroxy-3-indolyl)-piperidino)-ethyl]-8-chinolinsulfonamid.

### Beispiel 8

Analog Beispiel 1 erhält man durch Umsetzung von 1-(3-Amino-propyl)-4-(6-fluor-3-indolyl)-piperidin [erhältlich durch Umsetzung von 6-Fluorindol mit N-BOC-4-Chlor-piperidin, anschließende Abspaltung der Schutzgruppe, Umsetzung mit 1-Chlor-3-amino-propan] mit 1-Isochinolincarbon-säurechlorid ("C") in 200 ml Acetonitril nach üblicher Aufarbeitung das N-[3-(4-(6-Fluor-3-indolyl)-piperidino)-propyl]-1-isochinolincarboxamid, Dihydrochlorid-Tetrahydrat, Rf = 0,33 (Ethylacetat/Methanol 4 : 1; 1% Triethylamin).

Analog erhält man durch Umsetzung von "C"
mit 1-(3-Amino-propyl)-4-(4-fluor-indol-3-yl)-piperidin:
   N-[3-(4-(4-Fluor-3-indolyl)-piperidino)-propyl]-1-isochinolincarboxamid;
mit 1-(3-Amino-propyl)-4-(6-fluor-indol-3-yl)-piperidin:
   N-[3-(4-(6-Fluor-3-indolyl)-piperidino)-propyl]-1-isochinolincarboxamid;
mit 1-(3-Amino-propyl)-4-(5-methoxy-indol-3-yl)-piperidin:
   N-[3-(4-(5-Methoxy-3-indolyl)-piperidino)-propyl]-1-isochinolincarboxamid;
mit 1-(3-Amino-propyl)-4-(6-methoxy-indol-3-yl)-piperidin:
   N-[3-(4-(6-Methoxy-3-indolyl)-piperidino)-propyl]-1-isochinolincarboxamid;
mit 1-(3-Amino-propyl)-4-(5-trifluormethoxy-indol-3-yl)-piperidin:
   N-[3-(4-(5-Trifluormethoxy-3-indolyl)-piperidino)propyl]-1-isochinolincarboxamid;
mit 1-(3-Amino-propyl)-4-(4-Cyan-indol-3-yl)-piperidin:
   N-[3-(4-(4-Cyan-3-indolyl)-piperidino)-propyl]-1-isochnolincarboxamid;
mit 1-(3-Amino-propyl)-4-(6-Cyan-indol-3-yl)-piperidin:
   N-[3-(4-(6-Cyan-3-indolyl)-piperidino)-propyl]-1-isochinolincarboxamid;
mit 1-(3-Amino-propyl)-4-(5-cyan-indol-3-yl)-piperidin:
   N-[3-(4-(5-Cyan-3-indolyl)-piperidino)-propyl]-1-isochinolincarboxamid;
mit 1-(3-Amino-propyl)-4-(6-trifluormethyl-indol-3-yl)-piperidin:
   N-[3-(4-(6-Trifluormethyl-3-indolyl)-piperidino)-propyl]-1-isochinolincarboxamid;
mit 1 -(3-Amino-propyl)-4-(4-trifluormethyl-indol-3-yl)-piperidin:
   N-[3-(4-(4-Trifluormethyl-3-indolyl)-piperidino)-propyl]-1-isochinolincarboxamid;
mit 1-(3-Amino-propyl)-4-(5-methoxycarbonyl-indol-3-yl)-piperidin:
   N-[3-(4-(5-Methoxycarbonyl-3-indolyl)-piperidino)-propyl]-1-isochinolincarboxamid;
mit 1-(3-Amino-propyl)-4-(6-methoxycarbonyl-indol-3-yl)-piperidin:
   N-[3-(4-(6-Methoxycarbonyl-3-indolyl)-piperidino)-propyl]-1-isochinolincarboxamid;
mit 1-(3-Amino-propyl)-4-(4-methoxycarbonyl-indol-3-yl)-piperidin:
   N-[3-(4-(4-Methoxycarbonyl-3-indolyl)-piperidino)-propyl]-1-isochinolincarboxamid;
mit 1-(3-Amino-propyl)-4-(5-chlor-indol-3-yl)-piperidin:
   N-[3-(4-(5-Chlor-3-indolyl)-piperidino)-propyl]-1-isochinolincarboxamid;
mit 1-(3-Amino-propyl)-4-(6-chlor-indol-3-yl)-piperidin:
   N-[3-(4-(6-Chlor-3-indolyl)-piperidino)-propyl]-1-isochinolincarboxamid;
mit 1-(3-Amino-propyl)-4-(4-chlor-indol-3-yl)-piperidin:
   N-[3-(4-(4-Chlor-3-indolyl)-piperidino)-propyl]-1-isochinolincarboxamid.

### Beispiel 9

Analog Beispiel 1 erhält man durch Umsetzung von 1-(3-Amino-propyl)-4-(6-fluor-3-indolyl)-piperidin [erhältlich durch Umsetzung von 6-Fluorindol mit N-BOC-4-Chlor-piperidin, anschließende Abspaltung der Schutzgruppe, Umsetzung mit 1-Chlor-3-amino-propan] mit 6-Methoxy-chinolin-4-carbonsäurechlorid in 200 ml Acetonitril nach üblicher Aufarbeitung das N-[3-(4-(6-Fluor-3-indolyl)-piperidino)-propyl]-6-methoxychinolin-4-carboxamid, Dihydrochlorid-Hydrat, Rf = 0,33 (Ethylacetat/Methanol 4:1; 1% Triethylamin).

Analog erhält man durch Umsetzung von 4-Chinolincarbonsäurechlorid
mit 1-(3-Amino-propyl)-4-(4-fluor-indol-3-yl)-piperidin:
   N-[3-(4-(4-Fluor-3-indolyl)-piperidino)-propyl]-4-chinolincarboxamid;
mit 1 -(3-Amino-propyl)-4-(6-fluor-indol-3-yl)-piperidin:
   N-[3-(4-(6-Fluor-3-indolyl)-piperidino)-propyl]-4-chinolincarboxamid;
mit 1-(3-Amino-propyl)-4-(5-methoxy-indol-3-yl)-piperidin:
   N-[3-(4-(5-Methoxy-3-indolyl)-piperidino)-propyl]-4-chinolincarboxamid;
mit 1-(3-Amino-propyl)-4-(6-methoxy-indol-3-yl)-piperidin:
   N-[3-(4-(6-Methoxy-3-indolyl)-piperidino)-propyl]-4-chinolincarboxamid;
mit 1 -(3-Amino-propyl)-4-(5-trifluormethoxy-indol-3-yl)-piperidin:
   N-[3-(4-(5-Trifluormethoxy-3-indolyl)-piperidino)-propyl]-4-chinolincarboxamid;
mit 1-(3-Amino-propyl)-4-(4-Cyan-indol-3-yl)-piperidin:
   N-[3-(4-(4-Cyan-3-indolyl)-piperidino)-propyl]-4-chinolincarboxamid;
mit 1-(3-Amino-propyl)-4-(6-Cyan-indol-3-yl)-piperidin:
   N-[3-(4-(6-Cyan-3-indolyl)-piperidino)-propyl]-4-chinolincarboxamid;
mit 1-(3-Amino-propyl)-4-(5-cyan-indol-3-yl)-piperidin:
   N-[3-(4-(5-Cyan-3-indolyl)-piperidino)-propyl]-4-chinolincarboxamid;
mit 1 -(3-Amino-propyl)-4-(6-trifluormethyl-indol-3-yl)-piperidin:
   N-[3-(4-(6-Trifluormethyl-3-indolyl)-piperidino)-propyl]-4-chinolincarboxamid;
mit 1-(3-Amino-propyl)-4-(4-trifluormethyl-indol-3-yl)-piperidin:
   N-[3-(4-(4-Trifiuormethyl-3-indolyl)-piperidino)-propyl]-4-chinolincarboxamid;
mit 1-(3-Amino-propyl)-4-(5-methoxycarbonyl-indol-3-yl)-piperidin:
   N-[3-(4-(5-Methoxycarbonyl-3-indolyl)-piperidino)-propyl]-4-chinolincarboxamid;
mit 1-(3-Amino-propyl)-4-(6-methoxycarbonyl-indol-3-yl)-piperidin:
   N-[3-(4-(6-Methoxycarbonyl-3-indolyl)-piperidino)-propyl]-4-chinolincarboxamid;
mit 1 -(3-Amino-propyl)-4-(4-methoxycarbonyl-indol-3-yl)-piperidin:
   N-[3-(4-(4-Methoxycarbonyl-3-indolyl)-piperidino)-propyl]-4-chinolincarboxamid;
mit 1-(3-Amino-propyl)-4-(5-chlor-indol-3-yl)-piperidin:
   N-[3-(4-(5-Chlor-3-indolyl)-piperidino)-propyl]-4-chinolincarboxamid;
mit 1 -(3-Amino-propyl)-4-(6-chlor-indol-3-yl)-piperidin:
   N-[3-(4-(6-Chlor-3-indolyl)-piperidino)-propyl]-4-chinolincarboxamid;
mit 1-(3-Amino-propyl)-4-(4-chlor-indol-3-yl)-piperidin:
   N-[3-(4-(4-Chlor-3-indolyl)-piperidino)-propyl]-4-chinolincarboxamid.

Analog erhält man durch Umsetzung von 6-Methoxy-chinolin-4-carbonsäurechlorid
mit 1 -(3-Amino-propyl)-4-(4-fluor-indol-3-yl)-piperidin:
   N-[3-(4-(4-Fluor-3-indolyl)-piperidino)-propyl]-6-methoxy-4-chinolincarboxamid;
mit 1 -(3-Amino-propyl)-4-(6-fluor-indol-3-yl)-piperidin:
   N-[3-(4-(6-Fluor-3-indolyl)-piperidino)-propyl]-6-methoxy-4-chinolincarboxamid;
mit 1-(3-Amino-propyl)-4-(5-methoxy-indol-3-yl)-piperidin:
   N-[3-(4-(5-Methoxy-3-indolyl)-piperidino)-propyl]-6-methoxy-4-chinolincarboxamid;
mit 1-(3-Amino-propyl)-4-(6-methoxy-indol-3-yl)-piperidin:
   N-[3-(4-(6-Methoxy-3-indolyl)-piperidino)-propyl]-6-methoxy-4-chinolincarboxamid;
mit 1-(3-Amino-propyl)-4-(5-trifluormethoxy-indol-3-yl)-piperidin:
   N-[3-(4-(5-Trifluormethoxy-3-indolyl)-piperidino)-propyl]-6-methoxy-4-chinolincarboxamid;
mit 1-(3-Amino-propyl)-4-(4-Cyan-indol-3-yl)-piperidin:
   N-[3-(4-(4-Cyan-3-indolyl)-piperidino)-propyl]-6-methoxy-4-chinolincarboxamid;
mit 1 -(3-Amino-propyl)-4-(6-Cyan-indol-3-yl)-piperidin:
   N-[3-(4-(6-Cyan-3-indolyl)-piperidino)-propyl]-6-methoxy-4-chinolincarboxamid;
mit 1-(3-Amino-propyl)-4-(5-cyan-indol-3-yl)-piperidin:
   N-[3-(4-(5-Cyan-3-indolyl)-piperidino)-propyl]-6-methoxy-4-chinolincarboxamid;
mit 1-(3-Amino-propyl)-4-(6-trifluormethyl-indol-3-yl)-piperidin:
   N-[3-(4-(6-Trifluormethyl-3-indolyl)-piperidino)-propyl]-6-methoxy-4-chinolincarboxamid;
mit 1-(3-Amino-propyl)-4-(4-trifluormethyl-indol-3-yl)-piperidin:
   N-[3-(4-(4-Trifluormethyl-3-indolyl)-piperidino)-propyl]-6-methoxy-4-chinolincarboxamid;
mit 1-(3-Amino-propyl)-4-(5-methoxycarbonyl-indol-3-yl)-piperidin:
   N-[3-(4-(5-Methoxycarbonyl-3-indolyl)-piperidino)-propyl]-6-methoxy-4-chinolincarboxamid;
mit 1-(3-Amino-propyl)-4-(6-methoxycarbonyl-indol-3-yl)-piperidin:
   N-[3-(4-(6-Methoxycarbonyl-3-indolyl)-piperidino)-propyl]-6-methoxy-4-chinolincarboxamid;
mit 1-(3-Amino-prnpyl)-4-(4-methoxycarbonyl-indol-3-yl)-piperidin:
   N-[3-(4-(4-Methoxycarbonyl-3-indolyl)-piperidino)-propyl]-6-methoxy-4-chinolincarboxamid;
mit 1 -(3-Amino-propyl)-4-(5-chlor-indol-3-yl)-piperidin:
   N-[3-(4-(5-Chlor-3-indolyl)-piperidino)-propyl]-6-methoxy-4-chinolincarboxamid;
mit 1-(3-Amino-propyl)-4-(6-chlor-indol-3-yl)-piperidin:
   N-[3-(4-(6-Chlor-3-indolyl)-piperidino)-propyl]-6-methoxy-4-chinolincarboxamid;
mit 1 -(3-Amino-propyl)-4-(4-chlor-indol-3-yl)-piperidin:
   N-[3-(4-(4-Chlor-3-indolyl)-piperidino)-propyl]-6-methoxy-4-chinolincarboxamid.

### Beispiel 10

Man löst 1,2 g 2-(2-Chlorethoxy)-2,3dihydro-1H-benz[*de*]isochinolin-1,3-dion ("D") [erhältlich durch Umsetzung von N-Hydroxy-2,3-dihydro-1H-benz[*de*]isochinolin-1,3-dion mit 1,2-Dichlorethan zu 2-(2-Chlorethoxy)-2,3-dihydro-1 H-benz[de]isochinolin-1,3-dion] sowie 1,0 g 4-(5-Fluor-indol-3-yl)-piperidin [erhältlich durch Umsetzung von N-BOC-4-Chlor-piperidin mit 5-Fluor-indol und anschließende Abspaltung der Schutzgruppe] in 200 ml Acetonitril und rührt 8 Stunden bei Raumtemperatur. Nach üblicher Aufarbeitung erhält man 2-[2-(4-(5-Fluor-3-indolyl)piperidino)-ethoxy]-2,3-dihydro-1H-benz[de]isochinolin-1,3-dion, Hydrochlorid-Hydrat, F. 271°.

Analog erhält man durch Umsetzung von "D"
mit 4-(4-Fluor-indol-3-yl)-piperidin:
   2-[2-(4-(4-Fluor-3-indolyl)-piperidino)-ethoxy]-2,3-dihydro-1H-benz[*de*]isochinolin-1,3-dion, Hydrat, F. 162° (Zers.);
mit 4-(6-Fluor-indol-3-yl)-piperidin:
   2-[2-(4-(6-Fluor-3-indolyl)-piperidino)-ethoxy]-2,3-dihydro-1H-benz[*de*]isochinolin-1,3-dion, Hydrochlorid-Hydrat, F. 253°,
mit 4-(7-Ethyl-indol-3-yl)-piperidin:
   2-[2-(4-(7-Ethyl-3-indolyl)-piperidino)-ethoxy]-2,3-dihydro-1H-benz[*de*]isochinolin-1,3-dion, Hydrochlorid-Hemihydrat, F. 261°.

### Beispiel 11

Analog Beispiel 1 erhält man ausgehend von 1-(2-Aminoethyl)-4-(6-fluorindol-3-yl)-piperidin ("E") [erhältlich durch Umsetzung von N-BOC-4-Chlorpiperidin mit 6-Fluor-indol, anschließende Abspaltung der Schutzgruppe, Umsetzung mit 1-Chlor-2-amino-ethan] und 2-Naphthalincarbonsäurechlorid nach üblicher Aufarbeitung das N-[2-(4-(6-Fluor-3-indolyl)piperidino)-ethyl]-2-naphthalincarbonsäureamid, Hemihydrat, F. 194°.

Analog erhält man durch Umsetzung von "E"
mit 1-Naphthalincarbonsäurechlorid das
   N-[2-(4-(6-Fluor-3-indolyl)-piperidino)-ethyl]-1-naphthalincarbonsäureamid, Hydrochlorid-Sesquihydrat, F. 124°;
mit 2-Methyl-7-chlor-chinolin-4-carbonsäurechlorid das
   N-[2-(4-(6-Fluor-3-indolyl)-piperidino)-ethyl]-2-methyl-7-chlorchinolin-4-carbonsäureamid, Trihydrat, F. 206°;
mit 4-Methoxy-chinolin-2-carbonsäurechlorid das
   N[2-(4-(6-Fluor-3-indolyl)-piperidino)-ethyl]-4-methoxy-chinolin-2-carbonsäureamid, Dihydrochlorid-Dihydrat, F. 198°;
mit 3-Chinolin-carbonsäurechlorid das
   N[2-(4-(6-Fluor-3-indolyl)-piperidino)-ethyl]-3-chinolin-carbonsäureamid, Hydrat, F. 176°;
mit 2-Chinolin-carbonsäurechlorid das
   N-[2-(4-(6-Fluor-3-indolyl)-piperidino)-ethyl]-2-chinolin-carbonsäureamid, Diydrochlorid-dihydrat, F. 132,

Analog erhält man durch Umsetzung von 1-(3-Aminopropyl)-4-(6-fluorindol-3-yl)-piperidin
mit 2-Naphthalincarbonsäurechlorid das
   N[3-(4-(6-Fluor-3-indolyl)-piperidino)-propyl]-2-naphthalincarbonsäureamid, Hydrochlorid-Trihydrat, F. 102°;
mit 1-Naphthalincarbonsäurechlorid das
   N-[3-(4-(6-Fluor-3-indolyl)piperidino)propyl]-1-naphthalincarbonsäureamid, Hydrochlorid-Trihydrat, F. 85°;
mit 2-Methyl-7-chlor-chinolin-4-carbonsäurechlorid das
   N-[3-(4-(6-Fluor-3-indolyl)-piperidino)-propyl]-2-methyl-7-chlorchinolin-4-carbonsäureamid, Sesquihydrat, F. 89°;
mit 4-Methoxy-chinolin-2-carbonsäurechlorid das
   N-[3-(4-(6-Fluor-3-indolyl)-piperidino)-propyl]-4-methoxy-chinolin-2-carbonsäureamid, Dihydrochlorid-Dihydrat, F. 231°;
mit 3-Chinolin-carbonsäurechlorid das
   N-[3-(4-(6-Fluor-3-indolyl)-piperidino)-propyl]-3-chinolin-carbonsäureamid, Dihydrochlorid-Hemihydrat, F. 257°;
mit 2-Chinolin-carbonsäurechlorid das
   N-[3-(4-(6-Fluor-3-indolyl)-piperidino)-propyl]-2-chinolin-carbonsäureamid, Dihydrochlorid-Hydrat, F. 229.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 1 zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel 1, 9,38 g NaH₂PO₄^{.}2 H₂O, 28,48 g Na₂HPO₄ ^{.} 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Indolpiperidin-Derivate der Formel I 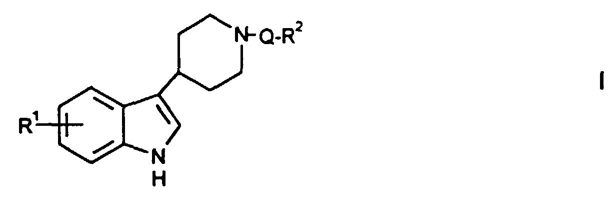 worin
R¹ H, A, OH, OA, F, Cl, Br, l, CF₃, OCF₃, CN, COOH, CONH₂, CONHA, CONA₂ oder COOA,
R² -NH-CO-Ar, -NH-SO₂-Ar oder D,
Q CₘH₂ₘ oder -O-CₙH₂ₙ
D 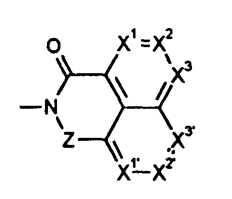
X¹_{,} X² und X³ sowie X^{1'}, X^{2'} und X^{3'} jeweils unabhängig voneinander N oder CH, wobei die jeweiligen H-Atome auch durch einen Substituenten, ausgewählt aus einer Gruppe bestehend aus A, OA, F, Cl, Br, l, CF₃, OCF₃, CN, COOH oder COOA ersetzt sein können,
Z CO, SO₂ oder SO,
A Alkyl mit 1 bis 6 C-Atomen,
Ar unsubstituiertes oder ein- oder zweifach durch A, OA, F, Cl, Br, I, CF₃, OCF₃, CN, COOH oder COOA substituiertes 1-Naphthyl, wobei ebenfalls eine, zwei, drei oder vier CH-Gruppen durch N ersetzt sein können,
m 1, 2, 3 oder 4 und
n 1, 2 oder 3
bedeuten,
sowie deren physiologisch unbedenkliche Salze.

2. (a) 2[2-(4-(5-Fluor-3-indolyl)-piperidino)-ethyl]-2,3-dihydro-1H-benz[*de*]isochinolin-1,3dion;
(b) 2-[2-(4-(6-Fluor-3-indolyl)-piperidino)-ethyl]-2,3-dihydro-1H-benz[*de*]isochinolin-1,3-dion;
(c) 2[2-(4-(4-Fluor-3-indolyl)-piperidino)-ethyl]-2,3-dihydro-1H-benz[*de*]isochinolin-1,3-dion;
(d) N[2-(4-(5-Fluor-3-indolyl)-piperidino)-ethyl]-8-chinolinsulfonamid;
(e) N[2-(4-(6-Fluor-3-indolyl)-piperidino)-ethyl]-8-chinolinsulfonamid;
(f) N[3-(4-(6-Fluor-3-indolyl)-piperidino)-propyl]-isochinolin-1-carboxamid;
(g) N[3-(4-(6-Fluor-3-indolyl)-piperidino)-propyl]-6-methoxychinolin-4-carboxamid,
und deren physiologisch unbedenkliche Salze.

3. Verfahren zur Herstellung von Indolpiperidin-Derivaten der Formel I gemäß Anspruch 1 sowie von deren Salzen, dadurch gekennzeichnet, daß man eine Verbindung der Formel II 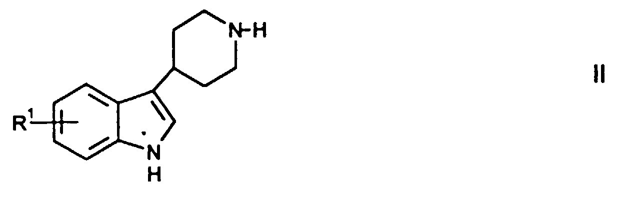 worin
R¹ die angegebene Bedeutung hat,
mit einer Verbindung der Formel III
R²-Q-L III
worin
L, sofern Q für -CₘH₂ₘ- steht Cl, Br, l, OH, O-CO-A', O-CO-Ph, O-SO₂-A', O-SO₂-Ph oder eine andere reaktionsfähig veresterte OH-Gruppe oder leicht nucleophil substituierbare Abgangsgruppe bedeutet und
L, sofern Q für -O-CₙH₂ₙ- steht H, -CO-A', -CO-Ph, -SO₂-A', -SO₂-Ph, wobei in beiden Fällen Ph für Phenyl oder Tolyl und A' für Alkyl oder Fluoralkyl stehen, oder eine andere reaktionsfähig veresterte OH-Gruppe oder leicht nucleophil substituierbare Abgangsgruppe bedeutet und
R² und Q die angegebenen Bedeutungen haben,
umsetzt,
oder daß man eine Verbindung der Formel IV 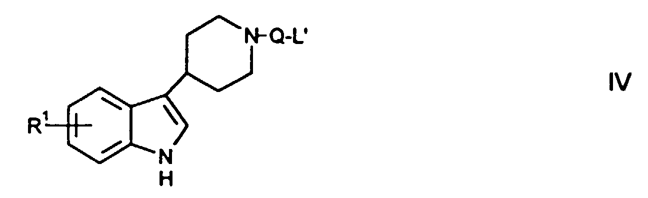 worin
L' Cl, Br, l, OH, O-CO-A', O-CO-Ph, O-SO₂-A', O-SO₂Ph, wobei Ph für Phenyl oder Tolyl und A' für Alkyl oder Fluoralkyl stehen, oder eine andere reaktionsfähig veresterte OH-Gruppe oder leicht nucleophil substituierbare Abgangsgruppe bedeutet und
R¹ und Q die angegebenen Bedeutungen haben,
mit einer primären oder sekundären Aminoverbindung der Formel V
R²-H V
worin
R² die angegebene Bedeutung hat,
umsetzt,
oder daß man eine Verbindung der Formel VI 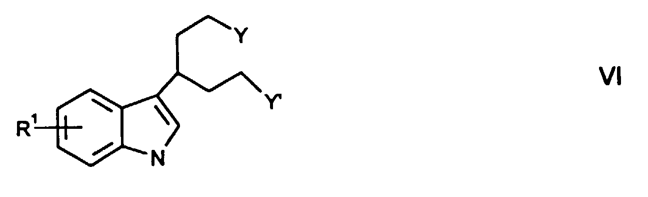 worin
Y und Y' gleich oder verschieden sein können und Cl, Br, l, OH, O-CO-A', O-CO-Ph, O-SO₂-A', O-SO₂-Ph, wobei Ph für Phenyl oder Tolyl und A' für Alkyl oder Fluoralkyl stehen, oder eine andere reaktionsfähig veresterte OH-Gruppe oder leicht nucleophil substituierbare Abgangsgruppe bedeutet und
R¹ die angegebene Bedeutung hat,
mit einem Amin der Formel VII
R²-Q-NH₂ VII
worin
R² und Q die angegebenen Bedeutungen haben,
umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls einen Rest R¹ in einen anderen Rest R¹ umwandelt, indem man z.B. eine OA-Gruppe unter Bildung einer OH-Gruppe spaltet und/oder eine CN-, COOH- oder COOA-Gruppe in eine andere funktionelle Gruppe umwandelt und/oder daß man eine erhaltene Base oder Säure der Formel I durch Behandeln mit einer Säure oder Base in eines ihrer Salze umwandelt.

4. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

7. Verwendung von Verbindungen der Formel I nach Patentanspruch 1 oder von deren physiologisch unbedenklichen Salzen bei Bekämpfung von Krankheiten.
